# EUROPEAN PATENT SPECIFICATION

(11) **EP 0 461 696 B1**
(45) Date of publication and mention of the grant of the patent: **15.09.1993**
(21) Application number: 91201319.0
(22) Date of filing: 31.05.1991
(51) Int. Cl.: C07K 7/38, A61K 37/40

(54) **Crystalline polypeptides with corticotropic activity**
Kristallinische Polypeptide mit corticotroper Aktivität
Polypeptides crystallines ayant une activité corticotropique

(30) Priority: 13.06.1990 EP 90201528
(43) Date of publication of application: 18.12.1991
(73) Proprietor: Akzo Nobel N.V., 6824 BM Arnhem (NL)
(72) Inventor: van Nispen, Johannes Wilhelmus Franciscus Maria, NL-5344 CE Oss (NL); van de Oetelaar, Petrus Johannes Maria, NL-5384 ET Heesch (NL); Janssen, Wilhelmus Petrus Andreas, NL-5344 JV Oss (NL)
(74) Representative: Hermans, Franciscus G.M.

(56) References cited:
- WO-A-82/03388
- JOURNAL OF CRYSTAL GROWTH, vol. 57, 1982 North Holland Publishing Co. pages 446-448
- G: ADMIRAAL ET AL: Crystallization of ACTH oligopeptides

## Description

### Background of the Invention

### Field

This invention relates to the preparation of a crystalline polypeptide, its use pharmacologically, pharmaceutical compositions containing the resulting compound, and the crystalline compound itself.

### State of the Art:

Oligopeptides such as the hexapeptide:

H-MetO₂-Glu-His-Phe-D-Lys-Phe-OH

are described in U.S. Patent No. 3,842,064 (as used herein, "MetO₂" is methionylsulfone; "Glu" is glutamyl; "His" is histidyl; "Phe" is phenylalanyl; and "D-Lys" is D-lysyl, if no special optical configuration is stated, then the L-form is intended). This patent and others (e.g. U.S. Patent Nos. 4,550,099, 4,104,371, and 4,110,322) disclose methods of producing this and similar polypeptides.

The disclosed methods of production and purification lead to a product comprising amorphous particles. This product, while it may be suitable for some types of administration (e.g. parenteral administration) is not as suitable for others (e.g. oral administration) due to a relatively low bioavailability. This low oral bioavailability of the product is probably due to its digestion in the gastrointestinal tract, its metabolism by the liver, or by both of these causes.

One attempt to purify these polypeptides is disclosed in the abstract, van Nispen et al, "Comparison of Several Approaches in the Solid Phase Synthesis of the Behaviourally Active Peptide H-Met(O₂)-Glu-His-Phe-D-Lys-Phe-OH," Seventh American Peptide Symposium Program and Abstracts (June 14-19, 1981, Univ. of Wisconsin-Madison). In this abstract, counter-current distribution (in fact, a multiple Craig distribution using, as a solvent system, 1-butanol-acetic acid-water (4-1-5, by volume)) was used after a "classical" solid phase preparation procedure using Boc amino acids to achieve a product containing a peptide component which was 95.9% of the hexapeptide.

With purification efforts to date, the resulting product is still comprised of amorphous particles, no matter how pure the product. These relatively pure, amorphous particles are still not well suited for certain routes of administration. Furthermore these amorphous particles might still generally contain certain undesirable solvent contaminants from the initial preparation and the purification steps, along with certain degradation products. A need remains for relatively pure hexapeptide suitable for convenient non-oral administration.

Methods of crystallizing compounds are described in Chase et al., Remington's Pharmaceutical Sciences, (16th ed., Mack Publishing Co., Easton. PA, U.S.A., 1980) ("Remington's"), at page 1535.

The difficulties encountered inn obtaining crystals of flexible molecules such as oligopeptides are described in Admiraal and Verweij "Crystallization of ACTH Oligopeptides", Journal of Crystal Growth, 57, pp. 446-448 (1982) where it took thousands of experiments to find reasonable crystallization conditions for various oligopeptides.

### Summary of the Invention.

The invention includes a method of obtaining an acid addition salt, crystalline compound of the formula:

H-MetO₂-Glu-His-Phe-D-Lys-Phe-OH.

This crystalline hexapeptide has surprisingly better intranasal absorption characteristics in comparison to comparably sized amorphous hexapeptide.

The method of obtaining the crystalline hexapeptide salt includes: a) Dissolving hexapeptide having a purity of at least 92% into an aqueous solvent, such as 10% aqueous acetic acid. b) Concentrating the resulting solution under reduced pressure at an elevated temperature. c) Measuring the pH of the concentrated solution. If the pH is between 4.5 and 4.8, then d) maintaining the temperature of the concentrated solution between 6 and 20° C. It the measured pH of the concentrated solution is however lower than 4.5, then adding water to the solution and repeating step b) until a pH of 4.5 to 4.8 is attained. Finally, e) after maintaining the temperature of the concentrated solution between 6 and 20° C, removing the crystals from the concentrated solution by filtration at lower temperatures. Optionally, the crystals may be f) washed with cold water and dried. A second "crop" of crystals may be obtained from the mother liquor. The entire process can be hastened and made more reliable by the addition of previously obtained "seed" crystals also comprising the acid addition salt of the hexapeptide.

Once obtained, the crystalline compound may be used in the manufacture of a medicament for the treatment of neuropathies, dementia, or both of these maladies. The invention thus includes a pharmaceutical composition, suitable for nasal administration, comprising the crystalline acid addition salt of the described hexapeptide (the "crystalline hexapeptide" as used herein). A sufficient amount of the crystalline hexapeptide may then be administered on a milligram per kilogram body mass basis, for a sufficient amount of time to decrease the neuropathies. In comparison to the amorphous particles, this crystalline hexapeptide is more rapidly and completely absorbed into systemic circulation through the nasal mucosa, without intestinal metabolism or the liver "first-pass effect".

In contrast with the treatment of animals, the crystalline hexapeptide-containing medicaments may also be used anticipatorily to prevent the occurrence of neuropathies. For example, a crystalline hexapeptide-containing medicament is administered, on a regular basis, to a mammal, such as a human, believed to be likely to suffer sometime in the future from neuropathies. A sufficient amount of compound is administered to the animal on a milligram per kilogram body mass basis, for a sufficient amount of time to prevent the neuropathy from occurring, thus preventing the need for treatment of the animal. Intranasal administration is generally a much more acceptable mode of administration to most patients than is parenteral administration.

### Description of the Preferred Embodiments

### I. The Hexapeptide-

Although the hexapeptides may be prepared by other methods known for the preparation of analogous compounds (e.g. by use of a solid phase synthesis), a method of making the hexapeptide is described in U.S. Patent No. 3,842,064, column 3, line 6 to column 6, line 15; column 7, line 1 to column 15, line 15; and EXAMPLE XI. During the process of preparation, solvents such as N,N-dimethylformamide (DMF), 1-butanol, 2-butanol, ethanol, methanol, ethyl acetate, methylene chloride, hexane, diethyl ether, water, acetic acid, and others may be used. Catalysts containing palladium or molybdenum may also be used in the preparation of the hexapeptide.

### II. Acid Addition Salts-

However made, the hexapeptide forms pharmacologically acceptable salts from pharmacologically acceptable organic and inorganic acids such as hydrochloric, hydrobromic, fumaric, phosphoric, ascorbic, tartaric, citric, lactic, maleic, palmitic, and other well-known acids. Especially preferred are the hydrochloric and acetic acid salts. The acid addition salts are obtained by reacting the hexapeptide with the acid.

### III. The Crystallization Process-

In the previously described process of obtaining crystalline hexapeptide salt, the choice of certain variables in the process are preferred.

For example, the compound containing the hexapeptide for crystallization preferably contains a peptide component comprising at least 92% of the hexapeptide. More preferred are those compounds containing 95% (as determined by HPLC) of the hexapeptide. Still more preferred are those compounds containing greater than 98% of the hexapeptide.

Purity of the peptide component of the resulting amorphous powder is preferably determined by HPLC, although other known techniques such as thin layer chromatography, HP ion exchange chromatography, or capillary zone electrophoresis may be used.

Preferred aqueous solvents for dissolving the hexapeptide-containing compound into include 10% aqueous acetic acid and hydrochloric acid.

Preferred temperatures for conducting the concentration step range between 30 and 40° C.

Preferred pressures for conducting the concentration step range between 15 and 30 mmHg.

The concentration of peptide in the aqueous solution will preferably be about 50, more preferably 52 % (wherein 10mg/ml =1%).

In one preferred embodiment, crystalline hexapeptide can also be obtained from a combination of water and water-miscible organic solvents which is added to the solution after the concentration step. In this process, the solution containing hexapeptide is concentrated, as before, at elevated temperature and reduced pressure until the concentration approaches 30 % (w/v) which will have a pH of 4.5 to 4.8. To the concentrated solution, a water-miscible organic solvent is added. Useful organic water-miscible solvents include ethanol, acetone, DMF, isopropanol, and t-butanol. Especially preferred are isopropanol and t-butanol.

Crystals obtained from the described process, vary in size from 0.05 to 1 millimeters, are white to off-white in color, have a melting point (decomposition) of 220° C (+/- 2° C) when the acid addition salt is acetate, and are monoclinic.

Preferably, previously obtained "seed" crystals are used to hasten the crystallization process. IV. Pharmaceutical Compositions-

Once the hexapeptide has been crystallized with its acid addition salt, it can be used to make a pharmaceutical composition. In its simplest form the pharmaceutical composition is merely the crystalline hexapeptide itself. Such a crystalline hexapeptide preferably contains 86 to 91 % (by perchloric acid titration) hexapeptide, from 6.0 to 10 % acetate, and from 1.6 to 4 % water.

This crystalline hexapeptide can be used to make numerous dosage forms such as powders for insufflation, powders for reconstitution, tablet triturates (e.g. dispensing tablets and hypodermic tablets), other tablets, and so forth.

The pharmaceutical compositions containing the crystalline hexapeptide are preferably dispensed in unit dosage forms, such as tablets, capsules, pills, powders, granules, suppositories, sterile parenteral solutions or suspensions and non-parenteral solutions or suspensions, containing suitable quantities of the pharmaceutically acceptable salt of the hexapeptide.

Methods and compositions for making such dosage forms are well-known to those skilled in the art. For example, methods of making powders, and their compositions are described at pages 1535 through 1552 of Remington's. Insufflations are described at page 1552, and insufflators are described at 1792. Methods and compositions for making tablets and pills, containing active ingredients, are described in Remington's, at pages 1553 through 1584. Methods of coating pharmaceutical dosage forms, and making prolonged release pharmaceuticals are described at pages 1585-1613 of Remington's. The contents of these pages are hereby incorporated by this reference.

The crystalline hexapeptide may also be incorporated into devices intended for implantation into a patient. Such devices, polymers intended for use therein, and methods of making both are described in U.S. Patent Nos. 3,773,919, 4,767,628, and 4,675,189. For example, a sufficient quantity of the crystalline hexapeptide could be incorporated into a PLAGA implant to allow for the release of hexapeptide (e.g. 5 mg per day for one month) into the patient's body.

One advantage with pharmaceutical compositions containing the crystalline versus the amorphous product, is that the pharmaceutical composition containing the crystalline salt product, having twice the bioavailability of the amorphous product, may need only contain half the absolute amount of the active ingredient on certain mucosa thus decreasing the amount of ingredient needed to be insufflated or otherwise administered and decreasing the ultimate cost of the composition. Such mucosa would include the nasal and the buccal mucosa.

Although the pharmaceutical compositions containing the crystalline hexapeptide may be formulated with adjuvants such as solubilizers, they need not be. The ability to use solely the crystalline hexapeptide (i.e. the crystalline acid addition salt of the hexapeptide) in a pharmaceutical composition to be applied to, for example, a nasal mucosa has advantages. For one thing, certain adjuvants are not suitable for chronic administration. However long term administration may be necessary for the particular person ingesting the hexapeptide. Another advantage is that the adjuvants necessarily take up a portion of the pharmaceutical composition, which portion may be better suited for the hexapeptide in order to decrease mucosal discomfort.

However if it is desired, suitable solubilizers, buffers, swelling agents, etc. may be used in such formulations. Buffering agents are preferably those which keep the hexapeptide in its unionized form.

### IV. Dosages-

The dosage of the crystalline acid addition salt / hexapeptide administered will generally be dependent upon the kind of disorder to be treated, the type of patient involved, his age, health, weight, kind of concurrent treatment, if any, and length and frequency of treatment.

The dosage forms will be administered over varying durations. To treat a disorder, the compounds are administered to a patient for a length of time sufficient to alleviate the symptoms associated with the disorders that the patient is suffering from. This time will vary, but periods of time exceeding two months are especially preferred. After the symptoms have been alleviated, the compound may then be discontinued to determine whether it is still required by the particular patient. Neuropathies which may be treated with the crystalline hexapeptide salt include traumatic neuropathies, neuropathies associated with leprosy and genetic disorders.

To prevent the occurrence of neuropathies, and thus alleviate the need for treatment, the compounds are administered to a person believed to be susceptible to suffering from neuropathies some time in the future (e.g. patients undergoing treatment with cytotoxic drugs such as vincristine; diabetics; alcoholics; etc.) for so long as he or she is believed susceptible. The length of such prophylactic administration of the compounds will of course vary, but again, periods of time exceeding two months are preferred. If the reason for the supposed susceptibility to a neuropathy has ceased to exist, the compound may then be discontinued. If however the reason for the disorder has not ceased to exist (e.g. in the case of diabetic neuropathies) the compound may be needed to be administered for the person's lifetime.

Illustratively, dosage levels of the administered active ingredients can be (intranasally): between 0.55 mg and 27 mg per day. In human therapy, daily doses of between 8 mg and 12 mg, administered intranasally, will preferably be used.

The invention is further explained by reference to the following examples.

### EXAMPLE I

### A. Preparation of the hexapeptide.

The hexapeptide may be conveniently obtained by the methods described in U.S. Patent No. 3,842,064 in EXAMPLE XI (1).

### B. Pretreatment of the crude hexapeptide.

If the hexapeptide obtained, from EXAMPLE I.A. or elsewhere, has a peptide component with purity of less than 92%, as measured by high-pressure liquid chromatography ("HPLC"), then the hexapeptide is preferably pretreated with H₂O-N,N-dimethyl formamide ("DMF"). The peptide is dissolved in water; the resulting solution optionally purified with charcoal; and the thus purified solution is added to DMF to yield an amorphous product.

### C. Crystallization of the hexapeptide.

4.7 grams (g) of hexapeptide having a peptide component with a purity of at least 92% as determined by HPLC were dissolved in 40 milliliters (ml) of 10% aqueous acetic acid by slight (30 - 40° centigrade (°C)) heating. The resulting solution was then concentrated under reduced pressure at 30 - 40° C to approximately 9 ml.

The pH of the concentrated solution was then measured. If the pH was in the range of 4.5 to 4.8, the concentrated solution was maintained at a temperature of between 6 and 20° C. If the measured pH was lower than 4.5, then 10 ml of water were added and the concentration step repeated until the proper pH range was attained.

After a few days, crystals were removed from the concentrated solution by filtration at lower (5 - 8°C) temperatures. The crystals were washed with ice cold water (approximately 1.8 ml) and dried. The yields ranged from 40 to 50% of hexapeptide / acid addition salt crystals. A second "crop" of crystals could be obtained from the mother liquor.

### D. Analysis of the hexapeptide crystals.

The crystals obtained from EXAMPLE I.C. were analyzed according to certain standard laboratory techniques.

The water content of the crystals (determined by the Karl Fischer method) was 1.8%. The crystals also contained 9.2% acetic acid as determined by gas chromatography. The free peptide content of the crystals was determined to be 88.5% as determined by perchloric acid titration.

High Performance liquid chromatography was used to analyze the peptide portion of the crystals in order to determine the purity of the hexapeptide. Although other devices will work, a Hewlett Packard (Palo Alto, CA, USA) 1090 M liquid chromatograph provided with a ternary solvent delivery system, an auto-injector and autosampler and a diode-array detector equipped with a computer work-station and printer / plotter facilities was used. The stationary phase consisted of a Supelcosil LC-18-DB, 25 cm X 4.6 mm I.D.; 5 µm particles (Supelco, Bellefonte, USA). The mobile phase was a gradient that consisted of A) a 0.5M NaH₂PO₄ with H₃PO₄ acid up to a pH of 2.1; B) water; and C) acetonitrile : water (60:40 v/v). The flow rate was 1.0 ml/minute at a temperature of 35°C. Detection was with ultraviolet light of 210 nm.

0.20 mg of the crystalline compound was dissolved in 1.00 ml of starting eluent (20%A-70%B-10%C). 100 microliters were injected into the HP1090M, and procedures were as indicated in the instruction manual. A 50 minute elution run was performed consisting of three minutes isocratic elution of 20%A-65%B-15%C followed by a linear gradient of 37 minutes to 20%A-60%B-20%C and an isocratic elution during the final ten minutes. The same procedure was followed with a sample of the amorphous hexapeptide.

The elution pattern of the run was recorded on the printer/plotter of the HP1090M. The retention times and peak areas of the main-component and of by-products that might have been present in the samples expressed as percentages of the total peak area under the elution curve were registered by the HP computer system.

Using this HPLC procedure, it was determined that the peptide portion of the crystals contained 99.3% of the hexapeptide. This purity compares with 98% purity attained by multiple, consecutive applications of prior art purification techniques to the amorphous compound.

The largest peptide portion (0.6 to 2.4%) of non-desired hexapeptide in the amorphous sample was a hexapeptide of the formula:

H-MetO₂-Glu-D-His-Phe-D-Lys-Phe-OH.

The crystalline salt product however contained less than 0.3% (as determined by HPLC) of this contaminant.

The crystalline salt product was further analyzed for the presence of DMF, 1-butanol, 2-butanol, ethanol, methanol, ethyl acetate, methylene chloride, hexane, diethyl ether, palladium, and molybdenum. Of these compounds, only traces of DMF (< 0.08% mass/mass) were found. In one crystalline sample, no DMF at all could be detected.

In contrast the hexapeptide "purified" by multiple, consecutive applications of the prior art techniques contained traces of ethanol, butanol, and DMF. The DMF was present in amounts as high as 0.4% mass/mass. In some samples of the hexapeptide purified by the prior art techniques, traces of molybdenum and palladium were also present, unlike in the crystalline salt product.

Thus the crystalline salt product is relatively more contaminant-free than the corresponding amorphous product, thus causing potentially fewer side-effects.

### EXAMPLE II

Crystalline hexapeptide can also be obtained from a combination of water and water-miscible organic solvents which solvents are added to the solution after the concentration step. To this end, the solution (20 ml) containing hexapeptide was concentrated to a 30 % (w/v) solution of the hexapeptide in 10% aqueous acetic acid (pH 4.2 to 4.8). Reagent grade isopropanol (5 ml) was mixed carefully with the solution. Crystals of the hexapeptide acid addition salt formed from this alternative method.

### EXAMPLE III

### A. Intranasal administration.

An animal study was designed to compare the bioavailability of the crystalline form of the hexapeptide (i.e. that obtained from EXAMPLE I.D., II or III) with the amorphous form of the hexapeptide (e.g. that obtained using counter-current distribution). Crystalline and amorphous forms of the hexapeptide were dosed intranasally to beagle dogs with a one-week wash out period between the dosages. Blood concentrations of the hexapeptide were measured by radio-immunoassay (RIA). The areas under the curves (AUC) of the two forms were calculated from the concentration data obtained after RIA using the trapezoidal rule. The bioavailabilities were calculated by comparing the AUC of the intranasal dosages. The bioavailabilities of the forms were calculated by comparing the AUC's of the intranasal ("i.n.") dosages with the intravenous ("i.v.") AUC (per dog) using the formula:

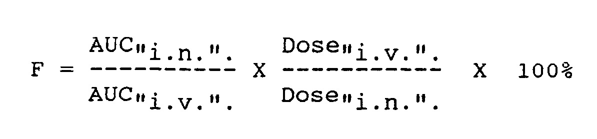

In the three dogs tested bioavailability of the crystalline form averaged a surprising 11.8% while bioavailability of the amorphous form averaged only 5.1%. Such increased absorption and bioavailability is unexpected since it is generally accepted that the amorphous form of a drug is more readily absorbed and hence more bioavailable than crystalline forms of a drug. See, e.g. E. Shefter, "Solubilization by solid-state manipulation", Techniques of Solubilization of Drugs, pp. 159-182 (Marcel Dekker, NY 1981). Reasons for the presumed superiority of amorphous over, for example, crystalline drug are that the irregular surface of an amorphous particle directly translates into greater surface area for comparably sized particles thus yielding an increased dissolution rate.

## Claims

1. A method of obtaining crystals of an acid addition salt of a hexapeptide of the formula:
H-MetO₂-Glu-His-Phe-D-Lys-Phe-OH
comprising the following steps:
a) dissolving said hexapeptide, having a purity of at least 92%, into an aqueous solution having a pH of 4 or less;
b) concentrating the resulting solution under reduced pressure at a temperature of about 28 to about 40° C;
c) measuring the pH of the concentrated solution;
d) if the pH of the concentrated solution is not between 4.5 and 4.8, then repeating steps b) and c), but if the pH is between 4.5 and 4.8, then
e) maintaining the temperature of the concentrated solution between 6 and 20° C for a period of time sufficient to form crystals.

2. A method of obtaining acetic acid addition salt crystals of a hexapeptide having the formula:
H-MetO₂-Glu-His-Phe-D-Lys-Phe-OH
comprising the following steps:
a) dissolving said hexapeptide having a purity of at least 92% into aqueous acetic acid;
b) concentrating the resulting solution under reduced pressure at a temperature of about 28 to about 40° centigrade;
c) measuring the pH of the concentrated solution;
d) if the pH of the concentrated solution is not between 4.5 and 4.8, then repeating steps b) and c), but if the pH is between 4.5 and 4.8, then
e) maintaining the temperature of the concentrated solution between 6 and 20° C for a period of time sufficient to form crystals.

3. The method according to claim 1 or claim 2, further comprising adding a water-miscible organic solvent to the concentrated solution.

4. The method according to claim 1 or claim 2, wherein said aqueous solvent is 10% aqueous acetic acid.

5. The method according to claim 1 or 2 further comprising adding crystals of said compound to the concentrated solution maintained at a temperature of between 6 and 20° C.

6. The method according to claim 1 wherein the hexapeptide dissolved into the aqueous solution comprises a peptide component containing at least 92% of the hexapeptide as determined by HPLC.

7. Crystalline acid addition salt of H-MetO₂-Glu-His-Phe-D-Lys-Phe-OH comprising:
from 86 to 91 %, by weight, of a peptide component
comprising H-MetO₂-Glu-His-Phe-D-Lys-Phe-OH, and
from 6.0 to 9.6 %, by weight, of an acid corresponding to the acid addition salt.

8. The crystalline acid addition salt of claim 7 further comprising from 1.6 to 3.2 %, by weight, of water.

9. The crystalline H-MetO₂-Glu-His-Phe-D-Lys-Phe-OH of claim 7 or claim 8 the peptide component consisting essentially of H-MetO₂-Glu-His-Phe-D-Lys-Phe-OH

10. A pharmaceutical composition comprising crystalline acid addition salt of H-MetO₂-Glu-His-Phe-D-Lys-Phe-OH.

## Claims (Claims for the following Contracting State(s): ES, GR)

1. A method of obtaining crystals of an acid addition salt of a hexapeptide of the formula:
H-MetO₂-Glu-His-Phe-D-Lys-Phe-OH
comprising the following steps:
a) dissolving said hexapeptide, having a purity of at least 92%, into an aqueous solution having a pH of 4 or less;
b) concentrating the resulting solution under reduced pressure at a temperature of about 28 to about 40° C;
c) measuring the pH of the concentrated solution;
d) if the pH of the concentrated solution is not between 4.5 and 4.8, then repeating steps b) and c), but if the pH is between 4.5 and 4.8, then
e) maintaining the temperature of the concentrated solution between 6 and 20° C for a period of time sufficient to form crystals.

2. A method of obtaining acetic acid addition salt crystals of a hexapeptide having the formula:
H-MetO₂-Glu-His-Phe-D-Lys-Phe-OH
comprising the following steps:
a) dissolving said hexapeptide having a purity of at least 92% into aqueous acetic acid;
b) concentrating the resulting solution under reduced pressure at a temperature of about 28 to about 40° centigrade;
c) measuring the pH of the concentrated solution;
d) if the pH of the concentrated solution is not between 4.5 and 4.8, then repeating steps b) and c), but if the pH is between 4.5 and 4.8, then
e) maintaining the temperature of the concentrated solution between 6 and 20° C for a period of time sufficient to form crystals.

3. The method according to claim 1 or claim 2, further comprising adding a water-miscible organic solvent to the concentrated solution.

4. The method according to claim 1 or claim 2, wherein said aqueous solvent is 10% aqueous acetic acid.

5. The method according to claim 1 or 2 further comprising adding crystals of said compound to the concentrated solution maintained at a temperature of between 6 and 20° C.

6. The method according to claim 1 wherein the hexapeptide dissolved into the aqueous solution comprises a peptide component containing at least 92% of the hexapeptide as determined by HPLC.

7. The method according to claim 1 wherein the crystalline acid addition salt of H-MetO₂-Glu-His-Phe-D-Lys-Phe-OH comprises from 86 to 91%, by weight, of a peptide component comprising H-MetO₂-Glu-His-Phe-D-Lys-Phe-OH, and from 6.0 to 9.6%, by weight, of an acid corresponding to the acid addition salt.

8. The method according to claim 7 wherein the crystalline acid addition salt further comprises from 1.6 to 3.2%, by weight, of water.

9. The method according to claim 7 or 8 wherein the peptide component of the crystalline acid addition salt consists essentially of H-MetO₂-Glu-His-Phe-D-Lys-Phe-OH.

## Patentansprüche

1. Ein Verfahren zur Gewinnung von Kristallen eines Säureadditionssalzes eines Hexapeptids der Formel
H-MetO₂-Glu-His-Phe-D-Lys-Phe-OH
welches folgende Stufen umfasst:
a) Auflösen des Hexapeptids, welches eine Reinheit von mindestens 92% aufweist, in einer wässrigen Lösung von pH = 4 oder weniger;
b) Konzentrieren der resultierenden Lösung unter vermindertem Druck bei einer Temperatur von etwa 28 bis etwa 40°C;
c) Messen des pH der konzentrierten Lösung;
d) Wiederholen der Stufen b) und c), falls der pH der konzentrierten Lösung nicht zwischen 4,5 und 4,8 liegt, aber wenn der pH zwischen 4,5 und 4,8 liegt,
e) Halten der Temperatur der konzentrierten Lösung auf einem Wert zwischen 6 und 20°C während einer genügend langen Zeit, um Kristalle zu bilden.

2. Ein Verfahren zur Gewinnung von Essigsäure-Additionssalz-Kristallen eines Hexapeptids der Formel
H-MetO₂-Glu-His-Phe-D-Lys-Phe-OH
welches folgende Stufen umfasst:
a) Auflösen des Hexapeptids, welches eine Reinheit von mindestens 92% aufweist, in wässriger Essigsäure;
b) Konzentrieren der resultierenden Lösung unter vermindertem Druck bei einer Temperatur von etwa 28 bis etwa 40°C;
c) Messen des pH der konzentrierten Lösung;
d) Wiederholen der Stufen b) und c), falls der pH der konzentrierten Lösung nicht zwischen 4,5 und 4,8 liegt, aber falls der pH zwischen 4,5 und 4,8 liegt,
e) Halten der Temperatur der konzentrierten Lösung auf einem Wert zwischen 6 und 20°C während genügend langer Zeit, um Kristalle zu bilden.

3. Das Verfahren nach Anspruch 1 oder 2, welches ferner den Zusatz eines mit Wasser mischbaren, organischen Lösungsmittels zur konzentrierten Lösung umfasst.

4. Das Verfahren nach Anspruch 1 oder 2, in welchem das wässrige Lösungsmittel 10%ige wässrige Essigsäure ist.

5. Das Verfahren nach Anspruch 1 oder 2, welches ferner den Zusatz von Kristallen dieser Verbindung zu der auf einer Temperatur zwischen 6 und 20°C gehaltenen konzentrierten Lösung umfasst.

6. Das Verfahren nach Anspruch 1, in welchem das in der wässrigen Lösung gelöste Hexapeptid eine Peptidkomponente umfasst, die mindestens 92% des Hexapeptids enthält, wie durch HPLC ermittelt.

7. Kristallines Säureadditionssalz von H-MetO₂-Glu-His-Phe-D-Lys-Phe-OH, welches 86 bis 91 Gewichtsprozent einer Peptidkomponente, umfassend H-MetO₂-Glu-His-Phe-D-Lys-Phe-OH, und 6,0 bis 9,6 Gewichtsprozent einer dem Säureadditionssalz entsprechenden Säure enthält.

8. Das kristalline Säureadditionssalz nach Anspruch 7, welches ferner 1,6 bis 3,2 Gewichtsprozent Wasser enthält.

9. Das kristalline H-MetO₂-Glu-His-Phe-D-Lys-Phe-OH-Salz nach Anspruch 7 oder 8, in welchem die Peptidkomponente im wesentlichen aus H-MetO₂-Glu-His-Phe-D-Lys-Phe-OH besteht.

10. Eine pharmazeutische Zusammensetzung, welche kristallines Säureadditionssalz von H-MetO₂-Glu-His-Phe-D-Lys -Phe-OH umfasst.

## Patentansprüche (Patentansprüche für folgende(n) Vertragsstaat(en): ES, GR)

1. Ein Verfahren zur Gewinnung von Kristallen eines Säureadditionssalzes eines Hexapeptids der Formel
H-MetO₂-Glu-His-Phe-D-Lys-Phe-OH
welches folgende Stufen umfasst:
a) Auflösen des Hexapeptids, welches eine Reinheit von mindestens 92% aufweist, in einer wässrigen Lösung von pH = 4 oder weniger;
b) Konzentrieren der resultierenden Lösung unter vermindertem Druck bei einer Temperatur von etwa 28 bis etwa 40°C;
c) Messen des pH der konzentrierten Lösung;
d) Wiederholen der Stufen b) und c), falls der pH der konzentrierten Lösung nicht zwischen 4,5 und 4,8 liegt, aber wenn der pH zwischen 4,5 und 4,8 liegt,
e) Halten der Temperatur der konzentrierten Lösung auf einem Wert zwischen 6 und 20°C während einer genügend langen Zeit um Kristalle zu bilden.

2. Ein Verfahren zur Gewinnung von Essigsäure-Additionssalz-Kristallen eines Hexapeptids der Formel
H-MetO₂-Glu-His-Phe-D-Lys-Phe-OH
welches folgende Stufen umfasst:
a) Auflösen des Hexapeptids, welches eine Reinheit von mindestens 92% aufweist, in wässriger Essigsäure;
b) Konzentrieren der resultierenden Lösung unter vermindertem Druck bei einer Temperatur von etwa 28 bis etwa 40°C;
c) Messen des pH der konzentrierten Lösung;
d) Wiederholen der Stufen b) und c), falls der pH der konzentrierten Lösung nicht zwischen 4,5 und 4,8 liegt, aber falls der pH zwischen 4,5 und 4,8 liegt,
e) Halten der Temperatur der konzentrierten Lösung auf einem Wert zwischen 6 und 20°C während genügend langer Zeit, um Kristalle zu bilden.

3. Das Verfahren nach Anspruch 1 oder 2, welches ferner den Zusatz eines mit Wasser mischbaren, organischen Lösungsmittels zur konzentrierten Lösung umfasst.

4. Das Verfahren nach Anspruch 1 oder 2, in welchem das wässrige Lösungsmittel 10%ige wässrige Essigsäure ist.

5. Das Verfahren nach Anspruch 1 oder 2, welches ferner den Zusatz von Kristallen dieser Verbindung zu der auf einer Temperatur zwischen 6 und 20°C gehaltenen konzentrierten Lösung umfasst.

6. Das Verfahren nach Anspruch 1, in welchem das in der wässrigen Lösung gelöste Hexapeptid eine Peptidkomponente umfasst, die mindestens 92% des Hexapeptids enthält, wie durch HPLC ermittelt.

7. Verfahren nach Anspruch 1, in welchem das kristalline Säureadditionssalz von H-MetO₂-Glu-His-Phe-D-Lys-Phe -OH 86 bis 91 Gewichtsprozent einer Peptidkomponente, umfassend H-MetO₂-Glu-His-Phe-D-Lys-Phe-OH, und 6,0 bis 9,6 Gewichtsprozent einer dem Säureadditionssalz entsprechenden Säure enthält.

8. Das Verfahren nach Anspruch 7, in welchem das kristalline Säureadditionssalz ferner 1,6 bis 3,2 Gewichtsprozent Wasser enthält.

9. Das Verfahren nach Anspruch 7 oder 8, in welchem die Peptidkomponente des kristallinen Säureadditions-salzens im wesentlichen aus H-MetO₂-Glu-His-Phe-D-Lys-Phe-OH besteht.

## Revendications

1. Procédé de préparation de cristaux d'un sel d'addition d'acide d'un hexapeptide représenté par la formule:
H-MetO₂-Glu-His-Phe-D-Lys-Phe-OH
comprenant les étapes suivantes:
(a) la dissolution de cet hexapeptide, ayant une pureté d'au moins 92%, dans une solution aqueuse ayant un pH inférieur ou égal à 4;
(b) la concentration de la solution résultante sous pression réduite à une température comprises entre environ 28° et environ 40°C;
(c) la mesure du pH de la solution concentrée;
(d) si le pH de la solution concentrée n'est pas compris entre 4,5 et 4,8, la répétition des étapes (b) et (c), mais si le pH est compris entre 4,5 et 4,8; alors
(e) le maintien de la température de la solution concentrée entre 6° et 20°C pendant une période de temps suffisante pour former des cristaux.

2. Procédé de préparation de cristaux de sel d'addition d'acide acétique d'un hexapeptide représenté par la formule:
H-MetO₂-Glu-His-Phe-D-Lys-Phe-OH
comprenant les étapes suivantes:
(a) la dissolution de cet hexapeptide, ayant une pureté d'au moins 92%, dans de l'acide acétique aqueux;
(b) la concentration de la solution résultante sous pression réduite à une température comprise entre environ 28° et environ 40°C;
(c) la mesure du pH de la solution concentrée;
(d) si le pH de la solution concentrée n'est pas compris entre 4,5 et 4,8, la répétition des étapes (b) et (c), mais si le pH est compris entre 4,5 et 4,8; alors
(e) le maintien de la température de la solution concentrée entre 6° et 20°C pendant une période de temps suffisante pour former des cristaux.

3. Procédé selon les revendications 1 ou 2, caractérisé en ce qu'il comprend en plus l'addition à la solution concentrée d'un solvant organique miscible à l'eau.

4. Procédé selon les revendications 1 ou 2, caractérisé en ce que ce solvant aqueux est de l'acide acétique aqueux à 10%.

5. Procédé selon les revendications 1 ou 2, caractérisé en ce qu'il comprend en plus l'addition de cristaux de ce composé à la solution concentrée qui est maintenue à une température comprise entre 6° et 20°C.

6. Procédé selon la revendication 1, caractérisé en ce que l'hexapeptide dissous dans la solution aqueuse comprend un composant peptidique contenant au moins 92% de l'hexapeptide, comme cela est déterminé par HPLC.

7. Sel d'addition d'acide cristallin de H-MetO₂-Glu-His-Phe-D-Lys-Phe-OH comprenant:
- entre 86 et 91% en poids d'un composant peptidique comprenant du H-MetO₂-Glu-His-Phe-D-Lys-Phe-OH, et
- entre 6,0 et 9,6% en poids d'un acide correspondant au sel d'addition d'acide.

8. Sel d'addition d'acide cristallin selon la revendication 7, caractérisé en ce qu'il comprend en plus entre 1,6 et 3,2% en poids d'eau.

9. H-MetO₂-Glu-His-Phe-D-Lys-Phe-OH cristallin selon les revendications 7 ou 8, caractérisé en ce que le composant peptidique consiste essentiellement en H-MetO₂-Glu-His-Phe-D-Lys-Phe-OH.

10. Composition pharmaceutique comprenant un sel d'addition d'acide cristallin de H-MetO₂-Glu-His-Phe-D-Lys-Phe-OH.

## Revendications (Revendications pour l'(les) Etat(s) contractant(s) suivant(s): ES, GR)

1. Procédé de préparation de cristaux d'un sel d'addition d'acide d'un hexapeptide représenté par la formule:
H-MetO₂-Glu-His-Phe-D-Lys-Phe-OH
comprenant les étapes suivantes:
(a) la dissolution de cet hexapeptide, ayant une pureté d'au moins 92%, dans une solution aqueuse ayant un pH inférieur ou égal à 4;
(b) la concentration de la solution résultante sous pression réduite à une température comprise entre environ 28° et environ 40°C;
(c) la mesure du pH de la solution concentrée;
(d) si le pH de la solution concentrée n'est pas compris entre 4,5 et 4,8, la répétition des étapes (b) et (c), mais si le pH est compris entre 4,5 et 4,8; alors
(e) le maintien de la température de la solution concentrée entre 6° et 20°C pendant une période de temps suffisante pour former des cristaux.

2. Procédé de préparation de cristaux de sel d'addition d'acide acétique d'un hexapeptide représenté par la formule:
H-MetO₂-Glu-His-Phe-D-Lys-Phe-OH
comprenant les étapes suivantes:
(a) la dissolution de cet hexapeptide, ayant une pureté d'au moins 92%, dans de l'acide acétique aqueux;
(b) la concentration de la solution résultante sous pression réduite à une température comprise entre environ 28° et environ 40°C;
(c) la mesure du pH de la solution concentrée;
(d) si le pH de la solution concentrée n'est pas compris entre 4,5 et 4,8, la répétition des étapes (b) et (c), mais si le pH est compris entre 4,5 et 4,8; alors
(e) le maintien de la température de la solution concentrée entre 6° et 20°C pendant une période de temps suffisante pour former des cristaux.

3. Procédé selon les revendications 1 ou 2, caractérisé en ce qu'il comprend en plus l'addition à la solution concentrée d'un solvant organique miscible à l'eau.

4. Procédé selon les revendications 1 ou 2, caractérisé en ce que ce solvant aqueux est de l'acide acétique aqueux à 10%.

5. Procédé selon les revendications 1 ou 2, caractérisé en ce qu'il comprend de plus l'addition de cristaux de ce composé à la solution concentrée maintenue à une température comprise entre 6° et 20°C.

6. Procédé selon la revendication 1, caractérisé en ce que l'hexapeptide dissous dans la solution aqueuse comprend un composant peptidique contenant au moins 92% de l'hexapeptide, comme cela est déterminé par HPLC.

7. Procédé selon la revendication 1, caractérisé en ce que le sel d'addition d'acide cristallin de H-MetO₂-Glu-His-Phe-D-Lys-Phe-OH comprend entre 86 et 91% en poids d'un composant peptidique comprenant du H-MetO₂-Glu-His-Phe-D-Lys-Phe-OH, et entre 6,0 et 9,6% en poids d'un acide correspondant au sel d'addition d'acide.

8. Procédé selon la revendication 7, caractérisé en ce que le sel d'addition d'acide cristallin comprend de plus de 1,6 à 3,2% en poids d'eau.

9. Procédé selon les revendications 7 ou 8, caractérisé en ce que le composant peptidique du sel d'addition d'acide cristallin consiste essentiellement en H-MetO₂-Glu-His-Phe-D-Lys-Phe-OH.
